# EUROPEAN PATENT APPLICATION

(11) **EP 1 952 816 A1**
(43) Date of publication of application: **06.08.2008**
(21) Application number: 07001673.8
(22) Date of filing: 26.01.2007
(51) Int. Cl.: A61K 36/258, A61K 36/185, A61K 36/296, A61K 36/25, A61P 15/10

(54) **Multi-component herbal composition for the treatment of male erectile dysfunction**

(71) Applicant: The Jordanian Pharmaceutical Manufacturing Co., 11710 Naor (JO)
(72) Inventor: Badwan, Adnan, Dr., 11185 Amman (JO); Taha, Hashem, Dr., 11821 Amman (JO); Ginna, Nidal, Adel, Dr., 11152 Amman (JO)
(74) Representative: Winkler, Andreas Fritz Ernst

(57) **Abstract**

The present invention relates to a combination of herbal extracts to treat male sexual dysfunction. More precisely, the present invention relates to a combination of five different herbal extracts including Ginseng extract, Tongkat Ali extract, Epimedium extract, Gotu Kola extract and flower pollen extract to restore male erectile function. The composition has a promoting effect on penile erectility, so that it can be effectively used for the improvement of erectile dysfunction.

## Description

The present invention relates to a combination of herbal extracts to treat male sexual dysfunction. More precisely, the present invention relates to a combination of five different herbal extracts including Ginseng extract to restore male erectile function.

### BACKGROUND OF THE INVENTION

Traditional herbs have been a revolutionary breakthrough in the management of erectile dysfunction and have become known world-wide as an 'instant' treatment. The modem view of the management of erectile dysfunction subscribes to a single etiology, i.e. the mechanism of erection. Erectile dysfunction affects 50% of men ages 40-70 in the U.S.A and is considered an important public health problem by the National Institute of Health (NIH).

A large number of pharmacological agents are orally consumed and vasoactive agents inserted intraurethrally or injected intrapenially to regain good erection. Some natural herbal products have the ability to act as aphrodisiac and can be used as a remedy to help people in case of sexual dysfunction to restore erection. Modem phytochemicals have developed from traditional herbs. Phytochemicals focus their mechanism of healing action to the root cause, i.e. the inability to control the proper function of the whole body system. Hence phytochemicals manage erectile dysfunction in the frame of sexual dysfunction as a whole entity.

There are a number of factors which influence male sexual vitality during aging. Smoking is known to decline testosterone level and to reduce male sexual vitality. Other conditions that are known to reduce man's sexual performance are depression, excessive ingestion of alcohol, anti-hypertension drugs, and diseases such as diabetes, benign prostatic hypertrophy (BPH), cardio vascular diseases.

The combination below contains five different standardized herbal extracts. Every single extract have long historical and traditional uses of sexual dysfunction and every single extract have a well-known mechanism of action, approved efficacy and safety. This combination has the ability to treat most of the main causes of erectile dysfunction based on the mechanism of action for each component by having additive synergistic effect of the combination.

| Composition: | |
|---|---|
| - Ginseng extract | 12.27 % (5 % gensinosides) |
| - Tongkat Ali | 24.53 % (1:50) |
| - Epimedium extract | 6.13 % (50 % icariin) |
| - Gotu Kola extract | 4.9 % (40 % asiatic acid) |
| - Flower Pollen extract | 16.56 % (3.6 mg/25 mg alpha-amino acids, 0.2 mg/25 |
| | mg phytosterols) |

### a) Ginseng (panax quinquelotius)

Ginseng is stated to possess thymoleptic, sedative, demulcent and stomachic properties, and is reputed to be an aphrodisiac, the medicinal activity of ginseng appears to reside in a number of saponins termed ginsenosides (panaxosides).

In a clinical study conducted by Salvati G*. et al* in 1996, use of *Panax ginseng* extract showed an increase in spermatozoa number/ml and progressive oscillating motility, an increase in total plasma testosterone and free testosterone, dihydrotestosterone (DHT), follicle stimulating hormone (FSH) and luteinizing hormone (LH) levels, but a decrease in mean prolactin (PRL). It is suggested that ginsenosides may have an effect at different levels on the hypothalamus-pituitary-testis axis.¹

In another study, Choi YD et al have reported that the long-term administration of ginseng enhances erective capacity and that its action is mediated by endothelium-derived relaxing factor and peripheral neurophysiologic enhancement. ²

### b) Tongkat Ali (Eurycoma longifolia)

*Eurycomal longifolia* is also known as Tongkat Ali in Malaysia³. Tongkat Ali is primary used as a male aphrodisiac. Many animal studies, both in rats and mice, have found administration of *E. longifolia* extracts to increase sexual arousal, motivation and frequency of sexual activity. ⁴⁻⁶ A large variety of constants have been identified including quassinoids, canthin-6-one alkaloids, beta-carbolines, tirucallane-type triterpenes, squalene derivatives, and biphenylneolignans. Tongkat Ali is also reported to have a testosterone-increasing effect. ⁴

### c) Horny goat weed (Epimedium grandifolium)

Horny Goat Weed has been used as an aphrodisiac for hundreds of years, and research has also proven its effect on improving sexual desire and performance. *Epimedium* contains a compound known as icariin that helps stimulate the nerves throughout the body. Advanced studies reveal that this stimulation effect is particularly experienced in the genital area of the body ^{7,8}

### d) Gotu Kola (Centella asiatica)

The primary active constituents of gotu kola are saponins (also called tripenoids), which include asiaticoside, madecassoside and madasiatic acid ⁹. These saponins are associated with wound healing promotion and prevent excessive scare formation.¹⁰ Clinical trials have also shown that it can help those with chronic venous insuffiency.^{11,12} Studies have shown that *Centella asiatica* has antidepressant and antioxidant actives ^{13,14}

### e) Flower Pollen

Pollen is known to include a wide variety of nutrients, including vitamins, minerals, amino acids, RNA, and DNA building factors, plant hormones, antioxidant factors, enzymes, unsaturated fatty acids, prostaglandin precursors, phytosterols and more. The antioxidant activity of pollen can be used in the treatment of infertility associated with chronic prostatitis that results from oxygen free radicals. ¹⁵

### SUMMARY OF THE PRESENT INVENTION

It is an object of the present invention to overcome the problem concerning sexual dysfunction by providing an efficient medication for the treatment of male erectile dysfunctions. In order to achieve this object, the present invention provides as medication a combination of herbal extracts. This medication is a combination of five different herbal extracts to restore male erectile function. Each herbal component of the composition has an additive synergistic effect on the treatment. The composition of the five components has a promoting effect on penile erectility, so that it can be effectively used for the improvement of erectile dysfunction.

In its main embodiment, the present invention relates to a composition comprising the components Ginseng extract, Tonkgat Ali extract, Epemedium extract, Gotu Kola extract and Flower Pollen extract.

Another embodiment of the present invention concerns a method of producing a composition according to the present invention.

In one embodiment the present invention further relates to a composition for use as a medicament.

In another embodiment the present invention concerns the use of a composition according to the present invention for the manufacture of a medicament for the treatment of erectile dysfunction.

One embodiment of the present invention relates further to a pharmaceutical composition comprising a composition according to the present invention and to a pharmaceutical applicable carrier and/ or excipient.

In another embodiment the present invention concerns a method of producing a pharmaceutical composition.

### DETAILED DESCRIPTION OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purposes of the present invention, all references as cited herein are incorporated by reference in their entireties.

Preferably, the terms used herein are defined as described in "A multilingual glossary of biotechnological terms: (IUPAC Recommendations)", Leuenberger, H.G.W., Nagel, B. and Kölbl. H. eds. (1996), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step.

In a first aspect the present invention provides a composition comprising the components Ginseng extract, Tonkgat Ali extract, Epemedium extract, Gotu Kola extract and Flower Pollen extract.

In one embodiment of the composition according to the present invention the amount of Ginseng extract is between 50 and 150 mg.

In a preferred embodiment of the composition the amount of Ginseng extract is about 100 mg.

In another embodiment of the composition according to the present invention the amount of Tonkgat Ali extract is between 100 and 300 mg.

In a preferred embodiment of the composition the amount of Tonkgat Ali extract is about 200 mg.

In a further embodiment of the composition according to the present invention the amount of Epemedium extract is between 25 and 75 mg.

In a preferred embodiment of the composition the amount of Epemedium extract is about 50 mg.

In another embodiment of the composition according to the present invention the amount of Gotu Kola extract is between 20 and 60 mg.

In a preferred embodiment of the composition the amount of Gotu Kola extract is about 40 mg.

In another embodiment of the composition according to the present invention the amount of Flower Pollen extract is between 90 and 180 mg.

In a preferred embodiment of the composition the amount of Flower Pollen extract is about 135 mg.

In another aspect the present invention concerns a method of producing a composition according to the present invention by advising appropriate amounts of the extracts.

In another aspect the present invention relates to a composition according to the present invention for use as a medicament.

In a further aspect the present invention concerns the use of a composition according to the present invention for the manufacture of a medicament for the treatment of erectile dysfunction.

In one embodiment the use is for mammals.

In a preferred embodiment the mammal is a human.

In another aspect the present invention relates to a pharmaceutical composition comprising a composition according to the invention and a pharmaceutically acceptable carrier and/or excipient.

In a further aspect the present invention relates to a method of producing a pharmaceutical composition according to the invention by advising a composition according to any of claims 1 to 11 and a pharmaceutically acceptable carrier and/or excipient.

The term "pharmaceutical acceptable carrier", as used herein, means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil; cottonseed oil; safflower oil, sesame oil, olive oil, corn oil and soybean oil; glycols, such as propylene glycol; esters such as ethyl oleate and ethyl laurate; agar; buffering agents such as magnesium hydroxide and aluminium hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgement of the formulator.

The pharmaceutical composition of this invention can be formulated for oral administration in solid or liquid form, for parenteral injection or for rectal administration. The pharmaceutical composition can be administered to humans and other mammals orally, sublingually, rectally, pareneterally, intracisternally, intraurethrally, intraperitoneally, topically (as powder, ointment or drop), bucally or as an oral or nasal spray. The term "parenterally", as used herein, refers to modes of administration which include intravenous, intramuscular, intraperitoneal, subcutaneous, intraarticular injection and infusion.

Sublingual compositions can be an effective dosage form in treating sexual dysfunction and sublingual compositions are well documented in the literature. Traditional sublingual tablets are usually designed as water soluble, although less soluble tablets are possible. Time release sublingual medications are disclosed in U.S. Pat. No. 3,428,728.

Pharmaceutical compositions of this invention for parenteral injection comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like), suitable mixtures thereof, vegetable oils ( such as olive oil) and injectable organic esters such as ethyl oleate. Proper fluidity may be maintained, for example, by the use of a coating such as lecithin, by the maintenance of the required particle size in the case of dispersions, and by the use of surfactants.

These compositions may also contain adjuvants such as preservative agents, wetting agents, emulsifying agents, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars, sodium chloride and the like. Prolonged absorbtion of the injectable pharmaceutical form may be brought about by the use of agents delaying absorption, for example, aluminium monostearate and gelatin.

In some cases, in order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Suspensions, in addition to the active compounds, may contain suspending agents, as, for example, ethoxylated isostearyl alcohols, polyoxyethylene sorbitol and sorbitan esters, microcrystalline cellulose, aluminium metahydroxide, bentonite, agar-agar, tragacanth, and mixtures thereof.

If desired, and for more effective distribution, the composition can be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

A further aspect of the present invention concerns a method of producing a pharmaceutical composition according to the invention.

### BRIEF DESCRIPTION OF THE FIGURES

**Figure 1****:**

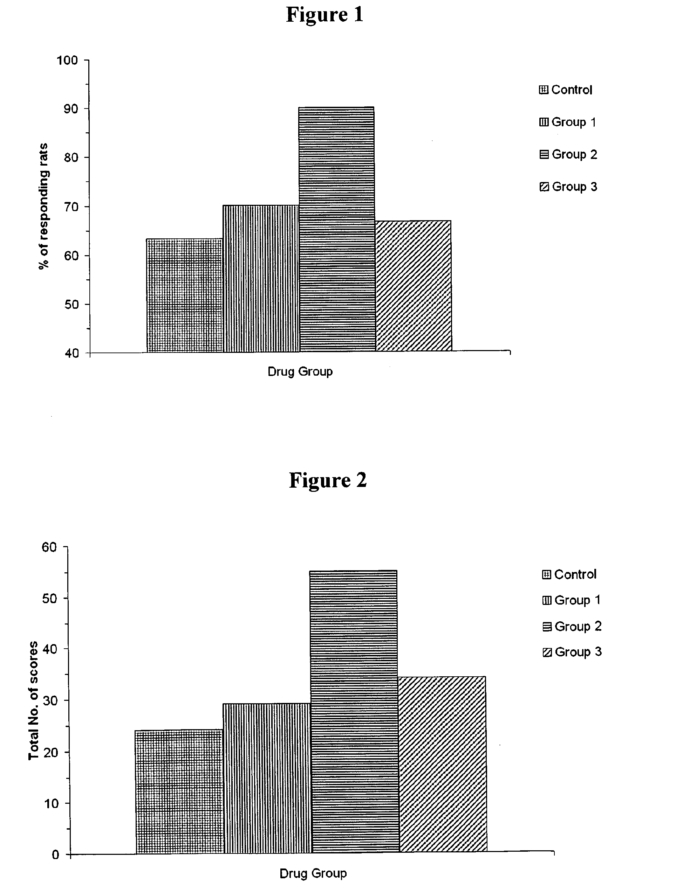

   Figure 1 shows the percentage of responding rats for each drug group along with the control. Each column represents the average of six experiments.
**Figure 2****:**
   Figure 2 shows the total number of scores recorded for each drug group after three days of treatment.
**Figure 3****:**

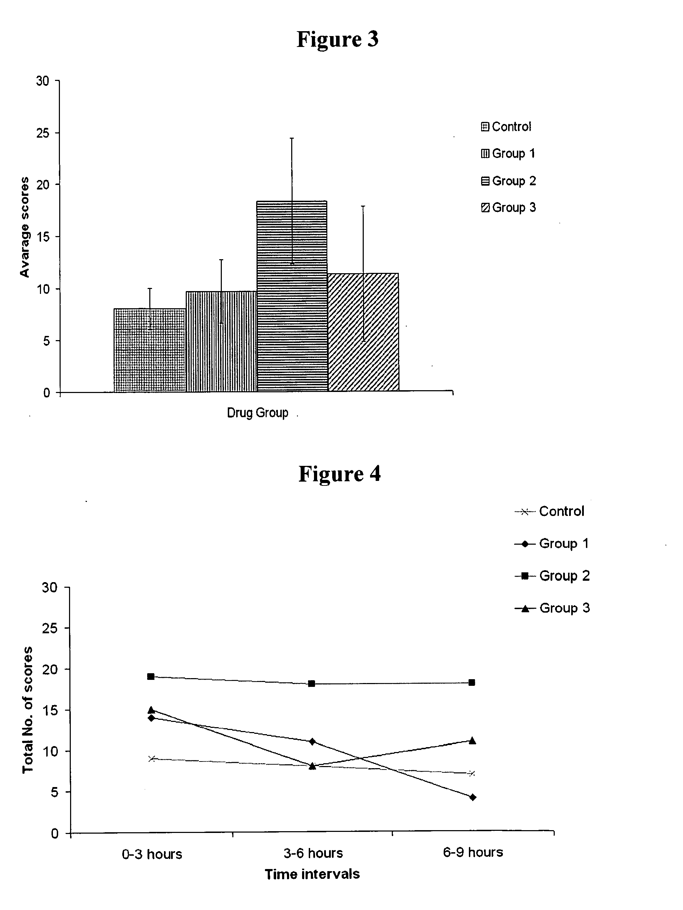

   Figure 3 shows the average of scores recorded after three days of drug administration for each drug group. Each bar represents the mean± standard deviation.
**Figure 4****:**
   Figure 4 shows the total number of scores reported at three hours dosing interval of six experiments for each group.
**Figure 5****:**

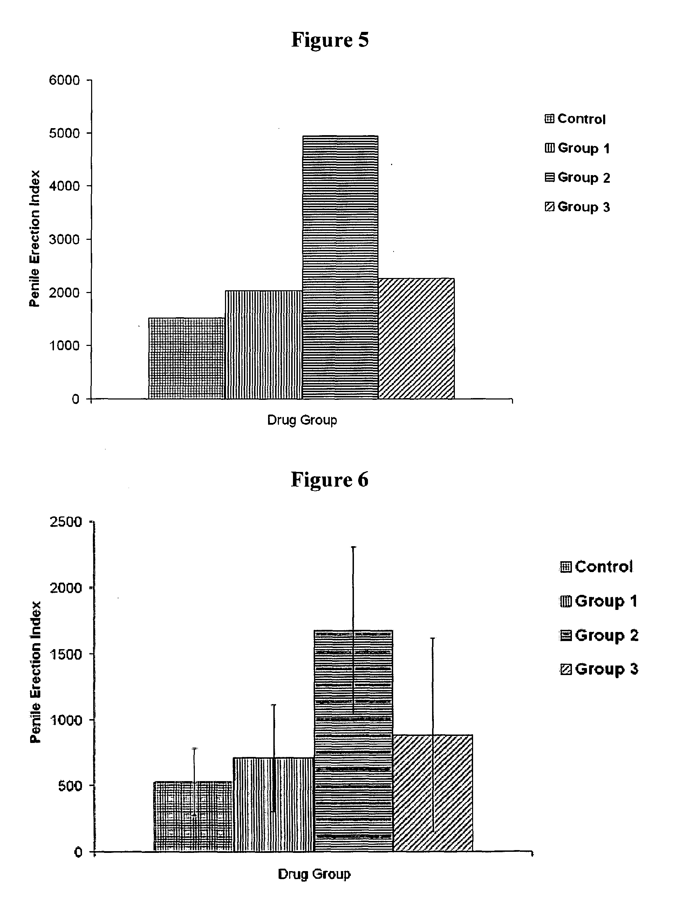

   Figure 5 shows the PEI (penile erection index) for different groups determined by multiplying the sum of all scores reported during three days of treatment by the average percent of responding rats.
**Figure 6****:**
   Figure 6 shows the PEI (penile erection index) for different groups determined by multiplying the average scores reported during three days of treatment by the average percent of responding rats. Each point represents the mean of six experiments ± standard deviation.
**Figure 7****:**

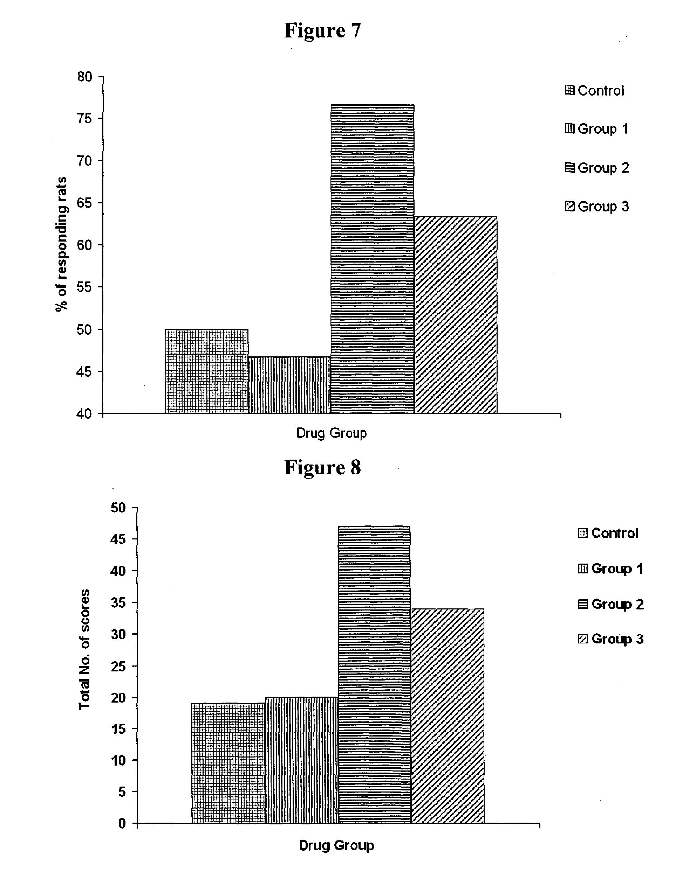

   Figure 7 shows the percentage of responding rats for each drug group along with the control. Each column represents the average of six experiments.
**Figure 8****:**
   Figure 8 shows the total number of scores recorded for each drug group after three days of treatment.
**Figure 9****:**

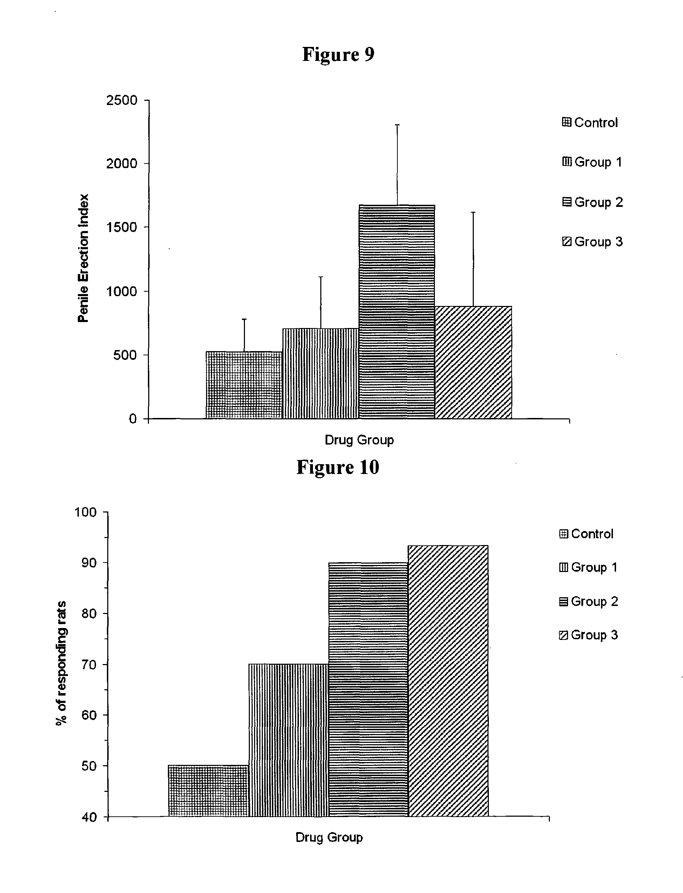

   Figure 9 shows the PEI (penile erection index) for different groups determined by multiplying the average scores reported during three days of treatment by the average percent of responding rats. Each point represents the mean of six experiments ± standard deviation.
**Figure 10****:**
   Figure 10 shows the percentage of responding rats for each drug group along with the control. Each column represents the average of six experiments.
**Figure 11****:**

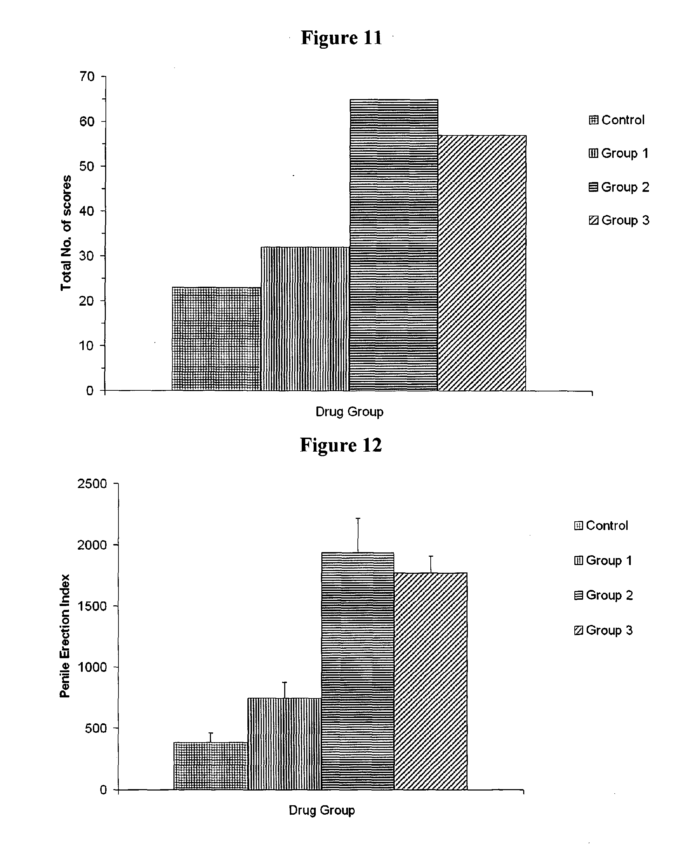

   Figure 11 shows the total number of scores recorded for each drug group after three days of treatment.
**Figure 12****:**
   Figure 12 shows the PEI (penile erection index) for different groups determined by multiplying the average scores reported during three days of treatment by the average percent of responding rats. Each point represents the mean of six experiments ± standard deviation.
**Figure 13****:**

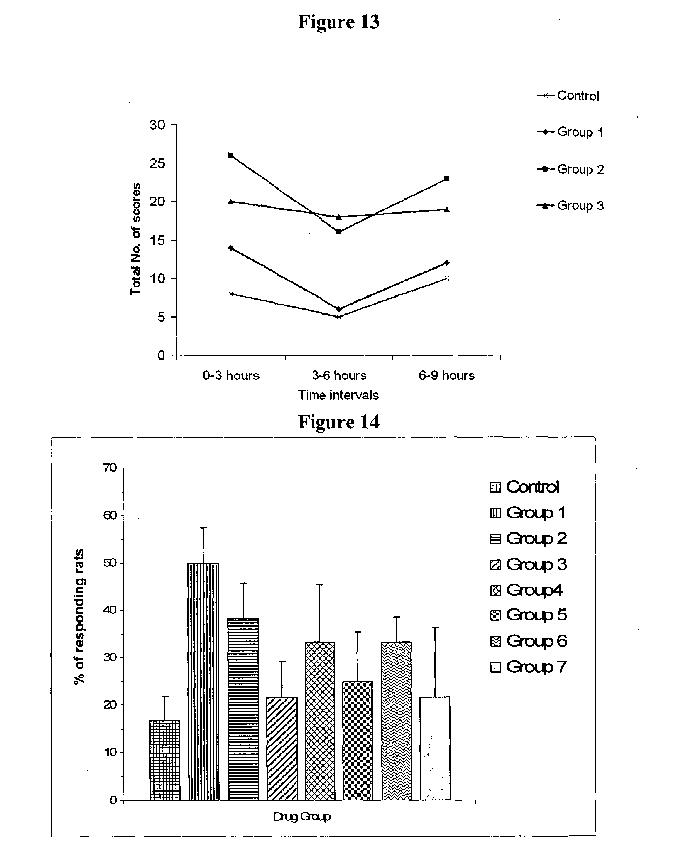

   Figure 13 shows the sum of scores reported at three hours dosing interval of six experiments for each group.
**Figure 14****:**
   Figure 14 shows the percentage of responding rats for each drug group along with the control. Each column represents the average of six experiments.
**Figure 15****:**

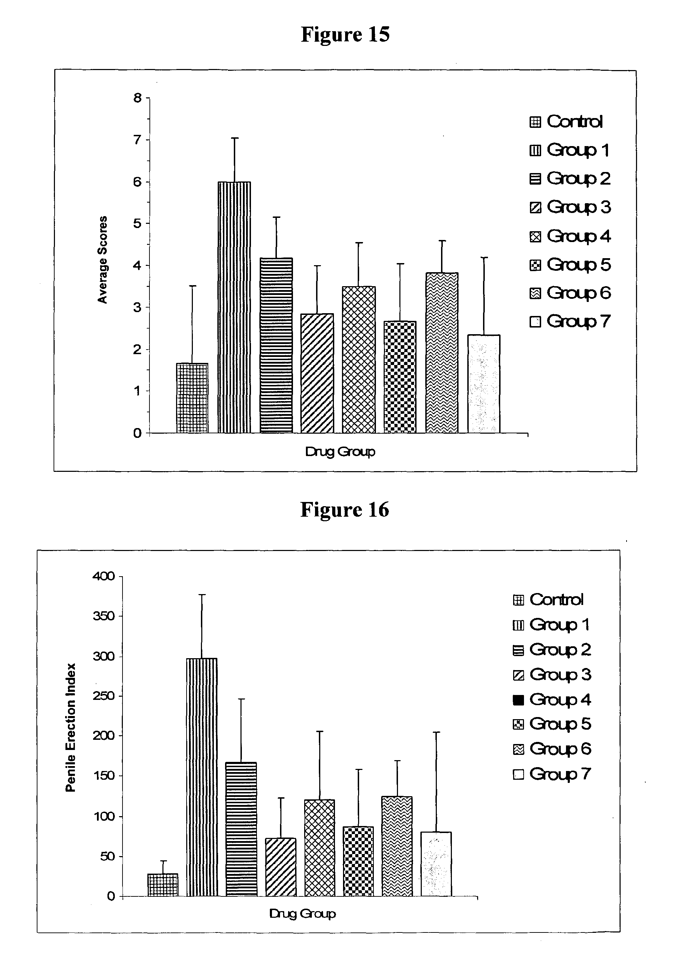

   Figure 15 shows the average of scores recorded after three days of drug administration for each drug group. Each bar represents the mean± standard deviation.
**Figure 16****:**
   Figure 16 shows the PEI (penile erection index) for different groups determined by multiplying the average scores reported during three days of treatment by the average percent of responding rats. Each point represents the mean of six experiments ± standard deviation.

### EXAMPLES

The following examples illustrate the present invention without, however, limiting the same thereto.

### Example 1:

### Preparation of composition with tablet preparation formula

**Table**

| **No.** | **Item** | **Quantities** | **Remarks** |
|---|---|---|---|
| 1. | Ginseng Extract | 100 mg | Active |
| 2. | Centella Asiatica Extract | 40 mg | Active |
| 3. | Eurycoma Longifolia | 200 mg | Active |
| 4. | Flower Pollen Extract | 135 mg | Active |
| 5. | Epemedium Extract | 50 mg | Active |
| 6. | Mycrocrystalline Celluose | 250 mg | Tablet Binder, Diluents |
| 7. | Cross Carmellose | 25 mg | Disintigrant |
| 8. | Aerosil | 5 mg | Glident |
| 9. | Magnisium Stearate | 10 mg | Lubricant |

### Tablet preparation method:

The different components, i.e. No. 1-5, see table above, were added together and mixed for 15 minutes. When the components No. 6 and 7 were added, the mixture was mixed for further 10 minutes. Then component No. 8 was added and the resulting mixture was again mixed for 10 minutes. In a final step component No. 9 was added and the obtaining mixture was thoroughly mixed for 5 minutes. At the end the mixture was compressed to a tablet.

### Example 2:

### The effect of multi-component herbal drugs on male rat sexual behavior

This study was conducted to reveal any sexual behavioral changes that emerge in male rats after the administration of different herbal formulas designed for the treatment of erectile dysfunction.
Three different formulas were examined and compared to a control group containing only vehicle. The following table (Table 1) describes the formulas used in this study along with the dose of administration. Each dose was based on 70kg human expected therapeutic dose to induce erection for such components.

**Table 1: Three formulas divided into three groups were studied on rat sexual behavior and compared to a fourth control group. The table shows the components of each formula and their corresponding weight per tablet.**

| **Group No.** | **Components** | **mg/tablet** | **Dose** |
|---|---|---|---|
| **Group 1** | Gingko biloba | 60 | 8 mg/kg |
| | Gotu Kola | 40 | |
| | Tongkat Ali | 270 | |
| | Epimedium | 50 | |
| | Flower pollen extract | 140 | |
| | | | |
| **Group 2** | Ginseng | 100 | 7.5 mg/kg |
| | Gotu Kola | 40 | |
| | Tongkat Ali | 200 | |
| | Epimedium | 50 | |
| | Flower pollen extract | 135 | |
| | | | |
| **Group 3** | Ginseng | 100 | 5 mg/kg |
| | Tongkat Ali | 200 | |
| | Epimedium | 50 | |
| | | | |
| **Group 4** | Control (D.W) | * | * |

Each animal group consisted from 10 Wistar rats weighing 200-300g. Each formula was dissolved freshly in an appropriate vehicle (distilled water) and doses were administered by oral gavage. Experiments were repeated three times using the same animals to reveal any changes in efficacy obtained from long drug administration. Control animals were given the vehicle alone without the drug. Rats were placed in an observation glass cages and were allowed free access to food and water. The doses were administered three times for each group. The animal groups were observed for three hours after each drug administration for penile erection, self-penile suction and possible copulation mounting. Number of rats responding to this experiment protocol was recorded along with the number of sexual activity episodes (self-sucking, erection or copulation movements). However, copulation-mounting behavior was not seen. Calculations of penile erection index (PEI) were performed for each group. PEI was calculated by multiplying the percentage of active rats (responding rats) by the total number of activity episodes (see e.g: Benassi-Benelli, F., Ferari, F., and Pellegrini Quaratotti, B., Arch. Int. Pharmacodyn., 242, 241-247 (1997), and Ang. H. H., Sim, M. K., Pharm. Sci., 3, 17-119 (1997) and references cited therein).

The outcome of this study shows clearly that the efficacy of Group 2 was distinguishable. 90% of tested rats responded to the medication in Group 2 compared to 70%, 67% and 63% responding rats in Group 1, Group 3 and the control respectively. (Figure 1)
The total number of scores obtained from each group during the whole study was calculated. The results again showed that group 2 induced the highest number of sexual episodes in the treated rats. A total of 55 scores were recorded for group 2 during three days of treatment where the average score was 18 compared to 10, 11 and 8 for group 1, 3 and the control respectively (Figure 2 and 3). Furthermore, another advantage observed with group 2 treatments is the consistency of reported episodes during the three dosing intervals (0-3, 3-6 and 6-9 hours time interval) compared to the stable reported control episodes. On the other hand, group 1 and 3 showed fluctuated effects reported during the same dosing intervals in comparison with group 2 and the control (Figure 4).

Penile erection index (PEI) for each group was determined in two ways. The first method considered the sum of all scores reported during three days of treatment multiplied by the average percent of responding rats (Figure 5) whereas the second method of PEI calculation considered the average of scores reported for each group during three days of treatment multiplied by the corresponding average percent of responding rats (Figure 6). In both ways, group 2 reported the highest PEI compared to the control group.

In conclusion, the formulas reported in this study induced sexual episodes in male rats in comparison with control groups. Animals in Group 2 showed the highest response to the used multi-component herbal treatment while minimal responses were seen in the other treatment groups.

### Example 3:

### Comparing different concentrations of Group 2 (Etana) on male rats sexual behavior

As seen in the first set of experiments, Group 2 formula (named Etana) showed a high PEI compared to the other formulas. In this experiment, different concentrations of Etana were tested as described in Table 2. The dose 7.5 mg/kg of Etana represents the recommended human expected therapeutic dose to induce male penile erection based on 70 kg human weight.

**Table 2: Three concentrations of Etana divided into three groups were studied on rat sexual behavior and compared to a fourth control group. The table shows the components of each formula and their corresponding weight per tablet along with the administered dose.**

| **Group No.** | **Components** | **mg/tablet** | **Dose** |
|---|---|---|---|
| **Group 1** | Ginseng | 100 | 2.5 mg/kg |
| | Gotu Kola | 40 | |
| | Tongkat Ali | 200 | |
| | Epimedium | 50 | |
| | Flower pollen extract | 135 | |
| | | | |
| **Group 2** | Ginseng | 100 | 7.5 mg/kg |
| | Gotu Kola | 40 | |
| | Tongkat Ali | 200 | |
| | Epimedium | 50 | |
| | Flower pollen extract | 135 | |
| | | | |
| **Group 3** | Ginseng | 100 | 15 mg/kg |
| | Gotu Kola | 40 | |
| | Tongkat Ali | 200 | |
| | Epimedium | 50 | |
| | Flower pollen extract | 135 | |
| | | | |
| **Group 4** | Control (D.W) | * | * |

The same experimental protocol was used in this experiment to determine the percentage of responding rats (Figure 7) and the total number of scores (Figure 8) achieved after three dose administrations in a three hours time intervals. Afterwards, the PEI (Figure 9) was calculated for each dose of Etana.

The results indicate that different concentrations of Etana produced a bell shape response where the maximum response was observed at the concentration of 7.5 mg/kg Etana which represents the recommended human therapeutic dose to achieve erection.

### Example 4:

### Comparing the effect of Etana on rat sexual behavior with Sildenafil Citrate as a positive control

In this set of experiments, the effect of Etana 7.5 mg/kg was compared to the effect of two therapeutic doses of Sildenafil citrate (Viagra ^{™}). Two other groups of rats in addition to Etana were given 0.71 mg/kg and 0.357 mg/kg Sildenafil which represents a dose of 50 and 25 mg/70 kg human recommended therapeutic dose of Sildenafil respectively (Table 3).

**Table 3: Two doses of Sildenafil Citrate in comparison to Etana were studied on rat sexual behavior and compared to a fourth control group. The table shows the components of each formula and their corresponding weight per tablet.**

| **Group No.** | **Components** | **mg/tablet** | **Dose** |
|---|---|---|---|
| **Group 1** | Sildenafil Citrate | 25 | 0.357 mg/kg |
| **Group 2** | Sildenafil Citrate | 50 | 0.71 mg/kg |
| **Group 3** | Ginseng | 100 | 7.5 mg/kg |
| | Gotu Kola | 40 | |
| | Tongkat Ali | 200 | |
| | Epimedium | 50 | |
| | Flower pollen extract | 135 | |
| **Group 4** | Control (D.W) | * | * |

The percentage of responding rats (Figure 10) and the total number of scores (Figure 11) achieved after three dose administrations in a three hours time intervals were determined for each group. Afterwards, the PEI (Figure 12) was calculated for all groups.

Etana 7.5mg/kg showed fewer efficacies than Sildenafil 0.71 mg/kg. But still the difference between the two PEI means is considered not statistically significant. On the other hand, Etana showed a high efficacy compared to the low dose of Sildenafil (0.57 mg/kg).

It is interesting to note that the number of scores achieved by Etana during the three dose intervals were stable compared to Sildenafil groups (both concentrations). There was a decline in the total number of scores reported after the second dose of Sildenafil followed by an increase in last dosing and monitoring interval (6-9 hours).

### Example 5:

### The effect of single component herbal extracts on male rats sexual behavior

The effect of single herbal extracts on inducing male rat erection is investigated in this experiment. All of Etana's components are investigated and compared to Etana multi component herbal extracts formula. Ginkgo biloba herbal extract was also investigated in this experiment. All of the groups were compared to a control containing only distilled water (Table 4).

**Table 4: Etana formula along with single herbal extracts were studied on rat sexual behavior and compared to a control group. The table shows the components of each formula and their corresponding weight per tablet.**

| **Group No.** | **Components** | **mg/tablet** | **Dose** |
|---|---|---|---|
| **Group 1** | Ginseng | 100 | 7.5 mg/kg |
| | Gotu Kola | 40 | |
| | Tongkat Ali | 200 | |
| | Epimedium | 50 | |
| | Flower pollen extract | 135 | |
| **Group 2** | Ginseng | 100 | 1.43 mg/kg |
| **Group 3** | Tongkat Ali | 200 | 2.68 mg/kg |
| **Group 4** | Gotu Kola | 40 | 0.57 mg/kg |
| **Group 5** | Epimedium | 50 | 0.71 mg/kg |
| **Group 6** | Flower pollen extract | 135 | 1.93 mg/kg |
| **Group 7** | Ginkgo biloba | 60 | 0.86 mg/kg |
| **Group 8** | Control (D.W) | * | * |

These set of experiments were conducted only on one monitoring time interval (3 hours). The percentage of responding rats (Figure 14) and the average number of scores (Figure 15) achieved after one-dose administrations were determined for each group by monitoring the groups for three hours. Afterwards, the PEI (Figure 16) was calculated for all groups.

It is clear from the results that Etana has a higher efficacy than any of its single components.

### References

1. Salvati G, Genovesi G, Marcellini L, Paolini P, De Nuccio I, Pape M, Re M. Effects of Panax Ginseng C.A Meyer saponions on male fertility. Panminerva Med 1996 Dec; 38(4): 249-54.
2. Choi YD, Rha KH , Choi HK. In vitro and in vivo experimental effect of Korean red ginseng on erection. J Urol. 1999 Oct; 162(4): 1508-11
3. Kuo PC, Shi LS, Damu AG, Su CR, Huang CH , Ke CH, Wu JB, Lin AJ, Bastow KF, Lee KH, Wu TS. Cytotoxic and antimalarial beta - carboline alkaloids from the roots of KH, Wu Ts. Cytoxoic and antimalarial beta - carboline alkaloids for the roots of Eurycoma longifoial. J Nat Prod. 2003 Oct; 66(10): 1324-7.
4. Ang HH, Lee KL Effect of Eurycoma longifolia Jack on orientaion activetis in middle aged male rats. Foundam Clin Pharmacol. 2002 Dec: 16(6): 479-83.
5. Ang HH, Cheang HS, Yusof AP. Effects of Eurcycoma longifolia Jack (Tongkat Ali) on the initiation of sexual performainse in inexperienced castretd male rats. Exp Anim. 2000 Jan, 49(1): 35-8.
6. Ang HH, Lee KL, Kiyoshi M. Eurycoma longifolia Jack enhances sexual motivation in middle - aged male mice, J Basic Clin Physol Pharmacol. 2003; 14(3): 301-8.
7. What is holy horny Goat weed PE from MDidea? Mechanism o Aciton How Does Horny Goat Weed and Icariiin Work? Get ready to heat us you love life with Icariin : Via Michael Derrida
8. Narrativ eHistoy and Mythology of Icarrin, Chapter3, Lover and Imaginaiton of Inter - Subjectivity. Via Michae Derrida. Published May 2003.
9. Kaming T. Clinical applicaion of Centella asiatica (L) Urb. In Herbs. Spices, and medicinal plants: Recent Advances in Botany, Horticulture, and Pharmacology, vol. 3 ., Craker LE, Simon JE(eds). Phoenxi, AZ: Oryx Press, 1986, 145-73
10. Morissset R, Cote NG, Panisset JC, ET AL. evaluation of the healing activity of hydrocotyle tincture in th tretment of wounds. Phytother Res 1987; 1:117-21.
11. Brinkhaus B, Linder M, Schuppan D, Hahn EG. Chemical, pharmacological and clinical profile of the East Asian medical plant Centella asitica. Phytomed 2000; 7:"427-48.
12. Pointell JP, Boccalon H, Cloarec M, et al. Titrated extract of Centella asiatica (TECA) in the treatment of venous insufficiency of the lower limbs. Angelology 1986; 37:420-1.
13. Chen Y, Han T, Qin L, Rui Y, Zheng H. Effect of total trierpens fro Centella asitica on the depression behavior and concentration of amino acid in forced swimming mice. Zhong Yao Cai. 2003 Dec: 26(12): 870-3
14. Jaswir I, Hasssan TH, said MZ. Ant oxidative Behavior of Malaysian plant extract in model and food oil systems. Asia Pac Jclin Nutr. 2004 Aug; 13 (Supp): S72.
15. Chen Hong - Jie, Wang Zhi - Ping, Chen Yi - Rong, Qin Da -Shan, Fu Sheng - Jun, Ma Bao- Liang, effects of Pollen extract EA-10, P 5 on chronic prostates or infertility with chronic prostitutes. Act of Pharmacology Sin 2002 Nov; 23 (11); 1035-1039.

## Claims

1. Composition comprising the components Gensing extract, Tonkgat Ali extract, Epemedium extract, Gotu Kola extract and Flower Pollen extract.

2. Composition according to claim 1, wherein the amount of Gensing extract is between 50 and 150 mg.

3. Composition according to claim 2, wherein the amount of Gensing extract is about 100 mg.

4. Composition according to any of claims 1 to 3, wherein the amount of Tonkgat Ali extract is between 100 and 300 mg.

5. Composition according to claim 4, wherein the amount of Tonkgat Ali extract is about 200 mg.

6. Composition according to any of claims 1 to 5, wherein the amount of Epemedium extract is between 25 and 75 mg.

7. Composition according to claim 6, wherein the amount of Epemedium extract is about 50 mg.

8. Composition according to any of claims 1 to 7, wherein the amount of Gotu Kola extract is between 20 and 60 mg.

9. Composition according to claim 8, wherein the amount of Gotu Kola extract is about 40 mg.

10. Composition according to any of claims 1 to 9, wherein the amount of Flower Pollen extract is between 90 and 180 mg.

11. Composition according to claim 10, wherein the amount of Flower Pollen extract is about 135 mg.

12. Method of producing a composition according to any of the preceding claims by advising appropriate amounts of the extracts.

13. Composition according to any of claims 1 to 11 for use as a medicament.

14. Use of a composition according to any of the preceding claims for the manufacture of a medicament for the treatment of erectile dysfunction.

15. Use according to claim 14 for mammals.

16. Use according to claim 15, wherein the mammal is a human.

17. Pharmaceutical composition comprising a composition according to any of claims 1 to 11 and a pharmaceutically acceptable carrier and/or excipient.

18. Method of producing a pharmaceutical composition according to claim 17 by advising a composition according to any of claims 1 to 11 and a pharmaceutically acceptable carrier and/or excipient.
